# EUROPEAN PATENT APPLICATION

(11) **EP 3 770 917 A1**
(43) Date of publication of application: **27.01.2021**
(21) Application number: 20177832.1
(22) Date of filing: 27.06.2014
(51) Int. Cl.: G16H 40/67, G16H 20/17, A61M 5/14

(54) **INFUSION PLANNING SYSTEM**

(30) Priority: 27.06.2013 US 201361840165 P
(62) Divisional of application: 14817611.8
(71) Applicant: Smiths Medical ASD, Inc., Rockland, MA 02370 (US)
(72) Inventor: ADAMS, Grant, Rockland, MA Massachusetts 02370 (US); WILKOWSKE, Eric, Rockland, MA Massachusetts 02370 (US); VAUGHAN, Mark, W., Rockland, MA Massachusetts 02370 (US)
(74) Representative: Harris, Oliver John Richard

(57) **Abstract**

An infusion planning system that improves timeliness and efficiency of patient care and includes methods, systems, and apparatuses for planning, visualizing, and coordinating medication delivery. These methods, systems and apparatuses can include improved scheduling capabilities for infusion pumps and other medical devices in hospitals or health care facilities. Some embodiments relate to improved recording and reporting of medical care information as well.

## Description

### RELATED APPLICATION

This application claims priority to U.S. Provisional Patent Application No. 61/840,165 filed June 27, 2013, which is incorporated herein by reference in its entirety.

### TECHNICAL FIELD

Embodiments relate generally to coordination of medical care and more particularly to planning, visualization, coordination, staffing, delivery and documentation tools for medical care, including management of patient infusion pumps, in hospitals and other medical care facilities.

### BACKGROUND

The demands of managing patient care within a hospital or medical facility are increasingly complex and can be challenging to coordinate accurately and efficiently. During the course of a day, a nurse or other medical caregiver or clinician might be responsible for the care of multiple patients, each of whom could receive medication from multiple infusion pumps. Therefore, the medical caregiver is burdened with keeping track of numerous historical, present, and planned future infusions. Further complicating the tracking of infusions is the fact that some infusions need to be coordinated with other care activities, such as blood draws and lab work, MRIs, CAT scans, nutritional intake, and other infusion therapies, for example.

Improved systems and methods are desired to make the jobs of medical caregivers easier and more efficient by providing these individuals with better understanding and management of the care they are providing to their patients. Therefore, improved methods and systems for scheduling, planning, coordinating and recording patient care are desired.

### SUMMARY OF THE INVENTION

Embodiments relate to infusion planning systems that improve planning and visualization of patient care and include methods, systems, and apparatuses for planning, visualizing, and coordinating medication delivery. These methods, systems and apparatuses can include improved scheduling capabilities for infusion pumps and other medical devices. The systems, methods, and apparatuses disclosed generally provide for improved planning in hospitals or health care facilities but also relate to improved recording and reporting of medical care information in various embodiments as well.

One embodiment relates to a non-transitory data storage media storing computer-usable instructions embodied thereon that cause computing devices to perform a method for scheduling infusion and medication delivery. The method can include receiving a plurality of medication event orders associated with one or more patients and creating a graphical user interface (GUI) for visualizing the orders. The medication event orders can include a plurality of medication infusion orders for administration by one or more infusion pumps. The medication infusion orders also can include delivery parameters for each medication infusion order. The method further includes assigning medication safety parameters to each medication infusion order, associating any related medication infusion orders and assigned medication safety parameters, and receiving patient information from a hospital information services database specific to the one or more patients.

The GUI can comprise a display for graphically and relatedly depicting a plurality of infusion events associated with one or more patients and set on a common scheduling timeline, each infusion event associated with a type of medication infusion order and including a representation of infusion amount and time, for example on corresponding vertical and horizontal axes or other first and second ordinates. Embodiments also can include display of an infusion delivery profile graphic for each infusion event that has an appearance that visually depicts the amount of infusion fluid for delivery and the time length of infusion for the medication infusion order associated with a corresponding horizontal infusion bar. Additionally, the graphical user interface can provide user manipulation of the infusion delivery profile graphic within the horizontal infusion bar to schedule the timing for a corresponding medication infusion.

Another embodiment of the invention is directed to a GUI for scheduling patient care events including scheduling a plurality of infusions delivered by one or more infusion pumps. The GUI includes a time schedule display including a plurality of vertical columns representing time intervals during a particular date or dates providing a chart having a horizontal timeline. The GUI also includes a plurality of horizontal infusion bars each representing an ordered infusion or medication for administration and an infusion delivery profile graphic associated with each ordered infusion which depicts both the amount of medication delivered to a patient and the length of time period needed for delivery. In the GUI, the infusion delivery profile graphic(s) are configured for movement within the horizontal infusion bar associated with the corresponding ordered infusion, such that the profile graphic is aligned with the vertical columns representing the time intervals over which the ordered infusion is scheduled for delivery.

A further embodiment of the invention relates to an infusion planning system for scheduling medical events including medical infusions. The system includes a control system including a processor, memory, and data bus. The control system receives a plurality of medication event orders associated with one or more patients including at least a plurality of medication infusion orders for administration by one or more infusion pumps including delivery parameters for each medication infusion order, assigns at least one medication safety parameter to each medication infusion order, associates any related medication infusion orders and assigned medication safety parameters with one another if so related, and receives patient information specific to the one or more patients from a hospital information services database. The infusion planning system further includes a medical caregiver device operatively coupled to the control system and a GUI. The GUI is displayed on the caregiver device containing a plurality of infusion delivery profile graphics corresponding to the plurality of medication infusion orders, each of the profile graphics presenting visual information corresponding to both a quantity of medication delivered and an amount of time for fluid delivery. The GUI further includes a schedule timeline permitting user manipulation of the infusion delivery profile graphics on the schedule timeline to assign and revise times for administration of the medication event orders.

A further embodiment includes a method for planning infusion treatments for a patient at a medical facility. The method includes accessing a database of timeline-based, graphical patient infusion records and filtering graphical patient infusion records with the aid of search software using parameters associated with a patient profile matching those of the patient to locate a set of relevant infusion timeline records. The method includes reviewing the set of relevant infusion timeline records to obtain information related to treatments of other individuals matching the patient profile as well as determining one or more patient infusion orders for the patient in view of the review of the set of relevant records. The method also includes submitting the one or more patient infusion orders electronically to an infusion planning system responsible for scheduling and recording infusion treatments for the patient within the medical facility.

### BRIEF DESCRIPTION OF THE DRAWINGS

The invention may be more completely understood in consideration of the following detailed description of various embodiments of the invention in connection with the accompanying drawings, in which:
Figures la-c are examples of scheduling environments showing management of various infusion orders and medical events with an infusion planning system, according to embodiments.
Figure 2 is an example of a graphical user interface of an infusion planning system, according to an embodiment.
Figure 3 is an example of a graphical user interface of an infusion planning system, according to an embodiment.
Figures 4a-b illustrate delivery of a pain drug as a function of time plotted on the same timeline as a fetal monitor strip, according to an embodiment.
Figure 5 is a block diagram of an infusion planning system utilizing a smart pump stack, according to an embodiment.
Figure 6 is a block diagram of an infusion planning system utilizing a smart pump stack, according to an embodiment.
Figure 7 is a block diagram of an exemplary infusion planning system computing environment, according to an embodiment.
Figure 8 is a block diagram of an exemplary infusion planning system computing environment, according to an embodiment.
Figure 9 is a block diagram of an exemplary infusion planning system computing environment, according to an embodiment.
Figure 10 is a block diagram of an exemplary infusion planning system computing environment, according to an embodiment.
Figure 11 is a flow diagram of an infusion planning method, according to an embodiment.
Figure 12 is a flow diagram of an infusion planning method, according to an embodiment.
Figure 13 illustrates the scalability of the display to suit various applications as well as the ability to filter and display infusion records based on a custom profile of parameters, according to an embodiment.
Figure 14 is a flow diagram of an infusion planning method, according to an embodiment.

The various embodiments may be embodied in other specific forms without departing from the essential attributes thereof, therefore, the illustrated embodiments should be considered in all respects as illustrative and not restrictive.

### DETAILED DESCRIPTION

Embodiments include an infusion and intake planning tool. Some embodiments will function as a day planner and scheduling tool for a medical caregiver such as nurse or other clinician. In such embodiments, this day planner will allow the medical caregiver to efficiently and effectively coordinate hospital care, especially with respect to delivery of medicament and fluids via an infusion pump whether for one patient receiving multiple treatments or multiple patients receiving single or multiple treatments.

### Infusion Planning System Examples

There are numerous environments and situations in which an infusion planner would be useful to a nurse or other medical caregiver or professional. Figures la-c represent a few examples of patient care arrangements in a hospital or medical facility that could require different types of planning and capabilities to appropriately coordinate care for ordered infusions or medical events. These arrangements generally depict infusions or medical events that require time periods to complete that are typically prescribed or known in advance.

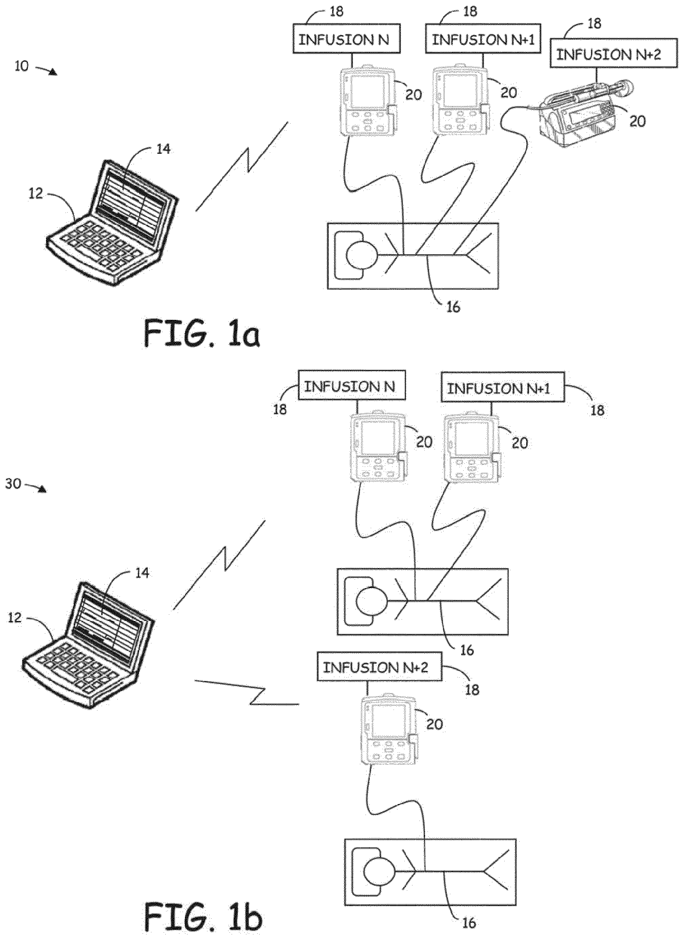

Figure 1a depicts an example of a scheduling environment 10 in which a medical caregiver device 12 displaying a graphical user interface (GUI) 14 is shown. The device 12 and GUI 14 are part of a larger scheduling/planning system that visually displays the timing of fluid delivery to a patient 16. The types of fluid delivery represented here include multiple infusions 18 from one or more infusion pumps 20. The medical caregiver device 12 can be a PC workstation of a nurse or clinician in a hospital. Alternatively, the medical caregiver device 12 can be a laptop, electronic tablet, smart phone, custom controller or other display and processing device providing a GUI 14. The infusion pumps 20 can be the same type or different types of pumps. Further, some types of infusion pumps 20 can be capable of delivering multiple infusions 18. The medical caregiver device 12 can operate autonomously from the pumps 20 in some embodiments, can be directly communicatively connected by wired or wireless connection to the pumps 20 themselves in some embodiments, or be communicatively coupled to a server or network in communication with the pumps 20 in other embodiments. The various system configurations will be discussed in greater detail below.

Similarly, Figure 1b depicts an example of a scheduling environment 30 in which a medical caregiver device 12 displaying a GUI 14 is shown as one part of a larger scheduling/planning system to visually display the timing of fluid delivery to multiple patients 16. The scheduling/planning system presents a display on which a medical caregiver can simultaneously visualize fluid delivery for multiple patients 16 each having one or more infusions 18 administered.

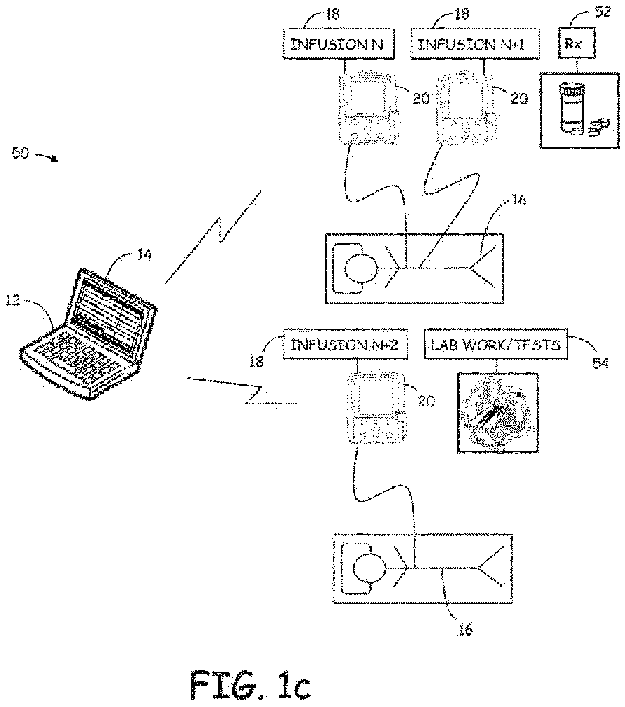

Figure 1c further shows an example of a scheduling environment 50 in which a medical caregiver device 12 displaying a GUI 14 is shown as one part of a larger scheduling/planning system to visually display the timing of fluid delivery to multiple patients 16, and further takes into account additional medication or medical events not necessarily related to medical infusion therapies. In such an embodiment, events including non-infusion medications 52 such as pills or oral medication, or lab work and test procedures 54 such as blood draws, lab work, MRIs, and CAT scans, can be implemented as part of the scheduling/planning system and GUI 14 of the caregiver device 12. As the timing of these types of medical events will restrict the scheduling of or make administration of infusion therapies at particular times more or less desirable, it is beneficial to visually account for such events when planning a particular schedule for a patient.

### Graphical User Interface (GUI)

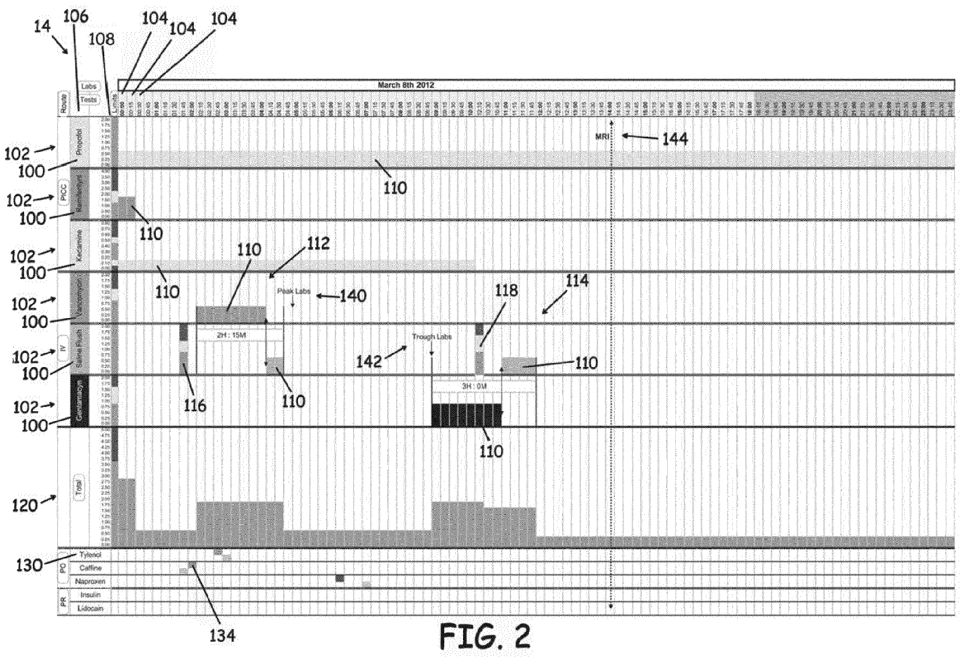

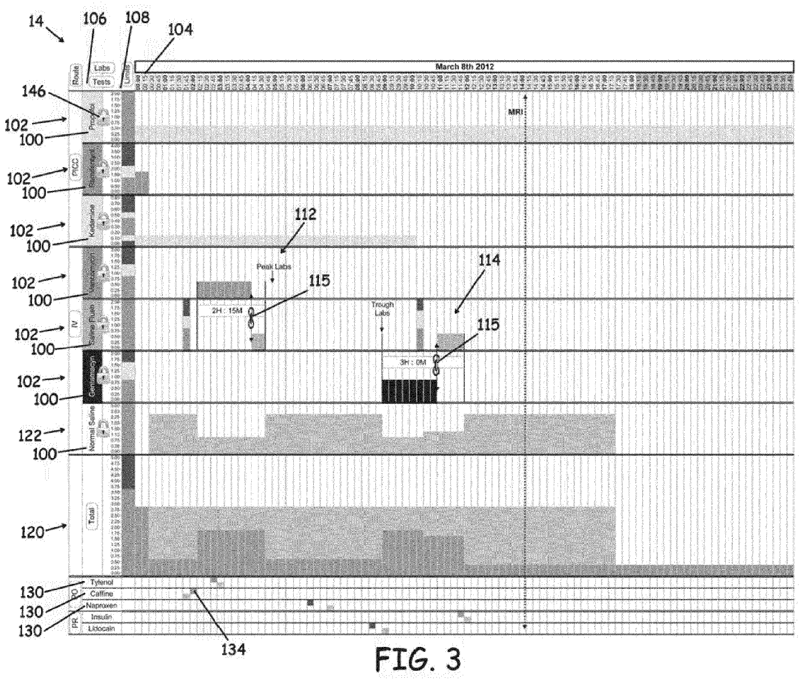

Figures 2 and 3 each show an embodiment of a GUI 14 comprising a display for an infusion planning system providing a visual interface in which multiple infusions and medical events are depicted as a function of time. Ordered infusion therapies 100 for delivery are represented by a plurality of horizontally disposed rows providing horizontal infusion bars 102 set against a timeline composed of vertical time columns 104 each representing a segment of time for a particular time of day on a particular date. In Figures 2 and 3, each of these vertical time columns 104 are shown to represent a fifteen minute period on March 8, 2012, for example. On the left side of the GUI 14, a column 106 which names the various infusion therapies is present in its own color coded segment. Accordingly, each respective infusion therapy 100 is listed within the corresponding horizontally disposed bar 102 for that infusion therapy 100. For example, in Figure 2 the named infusions 100 shown are Propofol, Remifentynl, Kedamine, Vancomycin, Saline Flush, and Gentamicin. Adjacent the column 106 of named infusions 100 is a color-coded scale 108 reflecting the acceptable range of fluid delivery amount for the various infusions. These ranges provide the medication safety limits for the respective infusion. To the right of each of the listed infusions 100 and color-coded scales 108 are infusion delivery profile graphics 110 for the various infusions 100. The infusion delivery profile graphics 110 comprise or include a series of generally adjacent bars of varying heights. The combination of these bars as an infusion delivery profile graphic 110 provides both time of fluid delivery information based upon the width of the combined bars and volume of fluid delivery information based on their height. These graphical representations help a nurse or medical caregiver to better visualize and plan for patient care, particularly when known or restricted amounts of time are required for infusions.

It is contemplated that in some embodiments the infusion bars 102 can be disposed in a direction that is non-horizontal as depicted in Figures 2 and 3. The axis scale representative of infusion amount is shown along a vertical first ordinate in Figures 2 and 3, although other orientations for this ordinate are possible as well. The axis scale representative of time is shown along a horizontal second ordinate in Figures 2 and 3, although other orientations for this ordinate are contemplated as well. The vertical disposition of the columns 104 in the figures depicting various time intervals should not be viewed as limiting the orientation or shape of such features. Moreover, GUIs having depictions of various orientations and shapes are contemplated by this disclosure.

At times, one or more infusions are linked with one another, such as in the case of an antibiotic like Vancomycin or Gentamicin which may require subsequent infusion of a saline flush after its infusion. Accordingly, these two infusions are linked together as the saline flush is used to push any remaining medication to the patient 16. These two linked infusion examples 112 and 114 are respectively depicted in both Figures 2 and 3. Figure 3 further includes a chain-link graphic 115 in GUI 14, as one optional way to further visually denote the linked relationship of infusions. This linking not only allows the scheduling of these infusions to be better understood and coordinated, but the linking also allows the medication safety limits for the earlier infusion (such as Vancomycin or Gentamicin in this example) to be applied to the subsequent saline flush. This can also be understood in a visual way as additional color coded limit bars 116 and 118 are respectively depicted within the saline flush infusion bar 102 timeline. A horizontal bar depicting the cumulative total of infused medication and fluid is set forth at total infusion bar 120.

While the timing of some infusions is scheduled to be fixed in time, other infusions are set up such that they are allowed to float. This is useful in a situation in which a patient, such as a neonate, has a fixed maximum volume of fluid that can be delivered at a time. A depiction of this can be understood from the GUI 14 in Figure 3. Here, the total value for volume of fluid in the total infusion bar 120 is fixed; however, the volume of Normal Saline at bar 122 is allowed to float such that the combined total of the infusion remains constant.

In Figures 2 and 3, medications are shown on the GUI 14 which are administered by non-infusion methods as well. This can include pills or oral medication for example. Horizontal bars 130 for these types of medication are provided at the bottom portion of the chart in Figures 2 and 3. Administration of these substances is not depicted with a graphical representation of their dosage or volume, as in the infusion profiles above, but simply with a colored marker 134 at the time of their administration. For example, orally administered Tylenol, Caffeine and Naproxen are represented by indications, like markers 134, at appropriate times within bars 130.

Additional useful, time-based events can be placed on the medication timeline as well. These can include various types of medical care events unrelated to infusions such as, for example, blood draws, lab work, MRIs, and CAT scans. In the examples shown in Figures 2 and 3, an arrow and label for a peak lab draw is shown at 140, an arrow and label for a trough lab draw is shown at 142, and an arrow and label for an MRI is shown at 144. The arrows or graphics depicted can extend across all other infusions, as in the case of the MRI indications 144, or can alternatively only be shown proximate those infusions 100 potentially related to that event, as with the peak and trough lab draws 140 and 142. These events are visually depicted so that staff can coordinate the full care of the patient timely and efficiently, especially with respect to infusions. For example, peak and trough measurements need to take a certain time with respect to the administration of the medication to help ensure the validity of the data obtained from the test. Likewise, knowing that a patient must be moved to perform an MRI allows a caregiver to schedule infusions such that the minimum number of infusions are running during the transport process.

In some embodiments, certain users can have the ability to lock the scheduled delivery of particular infusions 100 on one or more horizontal infusion bars 102 of the GUI 14. Infusions 100 that are locked can contain a visual graphic such as a padlock 146 adjacent the column 106 of corresponding named infusion 100. Accordingly, when an infusion 100 is locked, no graphical changes can be made to that horizontal infusion bar 102 until the infusion 100 is unlocked. This lock feature enables a user to more easily set and understand which infusions must or should occur at certain times so that only the remaining combinations of scheduled infusions can be changed. This allows more easily and effectively scheduled infusions and helps to prevent mistakes in rescheduling and planning of infusions at unwanted or unworkable times.

The GUIs 14 shown in Figures 2 and 3 are merely examples of embodiments of possible configurations and appearances for the type of scheduling device contemplated. Other arrangements for differently displaying infusions and medical events are contemplated as well. Other embodiments can display multiple patients receiving infusions in similar embodiments. GUI displays 14 that include a large plurality of patients and/or infusions can require a display that reduces the amount of infusion data viewable or is otherwise downwardly-scalable to accommodate the number of patients or infusions shown.

### Reporting and Recording

Some embodiments described above can be used for forward-looking planning of infusions and medical care events. However, other embodiments also include backward-looking or real-time reporting and recording of infusions and medical events as well.

In some embodiments, which include reporting or recoding of data, times when infusions or medications are ordered can be compared to the times at which these infusions or medications are actually delivered to the patient. One example of this can be seen at the bottom of Figures 2 and 3 where different colored markers 134 are indicated within the horizontal bars 130 for medication order times and delivery times.

In certain embodiments, a display including future planned infusions and events as well as recently-recorded actual infusions delivered are displayed on the same screen. In some embodiments this can be done using a split or tiled screen having a timeline extending horizontally across the screen where the right side of the screen depicts times and planned infusions in the future. Similarly, the left side of the screen depicts actual delivered infusions in the past. Accordingly, the vertical split between these two displays represents the present time that one is viewing the display. In such a display, the infusion delivery profile graphics 110 and other events will generally move from right to left across the display screen as time passes. Deviations from the projected infusions that are sensed and recorded when they actually occur in real-time may necessitate updates to the other projected infusions scheduled yet to occur. These updates can be prompted by the system in some embodiments and recommendations and advice for altering the schedule can be suggested in some embodiments. Some embodiments will require approval to certain modifications in scheduling by a physician or medical professional. Certain embodiments will enable modifications in scheduling to automatically occur when necessary.

In other embodiments, incorporating reporting or recording of actual data, additional patient monitoring information, such as the vital signs of a patient, can be included on the same screen as well. The combination of this medication administration information and patient information could provide useful medical records. For example, a physician could look at the infusion history of a specific drug and the vital parameter that that drug affects as a function of time. Patient monitoring information can be provided in real-time in various embodiments.

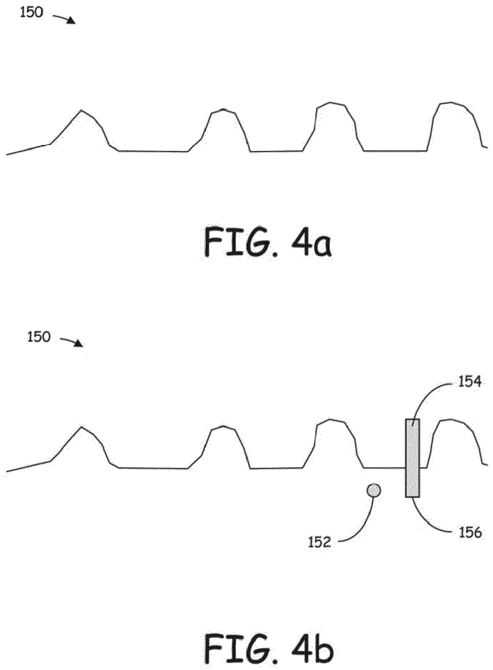

In another embodiment, the delivery of a pain drug as a function of time could be plotted on the same timeline as a fetal monitor strip. This would give the doctor the ability to see when the contractions are happening and help to close the loop on pain control as a function of contractions. Figure 4a depicts an example of a contraction strip chart 150 from a fetal monitor. Given the repetitive nature of contractions and a somewhat constant time period of occurrence, a learning algorithm could be applied to the Patient Controlled Analgesia (PCA) delivery with data feedback from the fetal monitor. For example, the expectant mother could push the PCA dose button to request a dose at 152, and rather than giving the dose at that time, the pump could delay the dose until the optimum time for a bolus 154 at time 156 prior to the next contraction. This delay would be based on how quickly the drug takes effect. For example, if a known drug takes three minutes to take full effect, the optimum time would be about three minutes prior to the next expected contraction. This is visually depicted in Figure 4b.

Patient data to base infusion delivery parameters can be used throughout a hospital. For example, in the Cardiac Intensive Care Unit (CICU), the delivery rate of vasoactives or vasopressors could be determined by the vital signs of a patient. The pump 20 could work with a system to alarm if the patient's vital reading falls outside of a specified band. The pump 20 could then automatically adjust to accommodate the change in vital signs.

### NICU

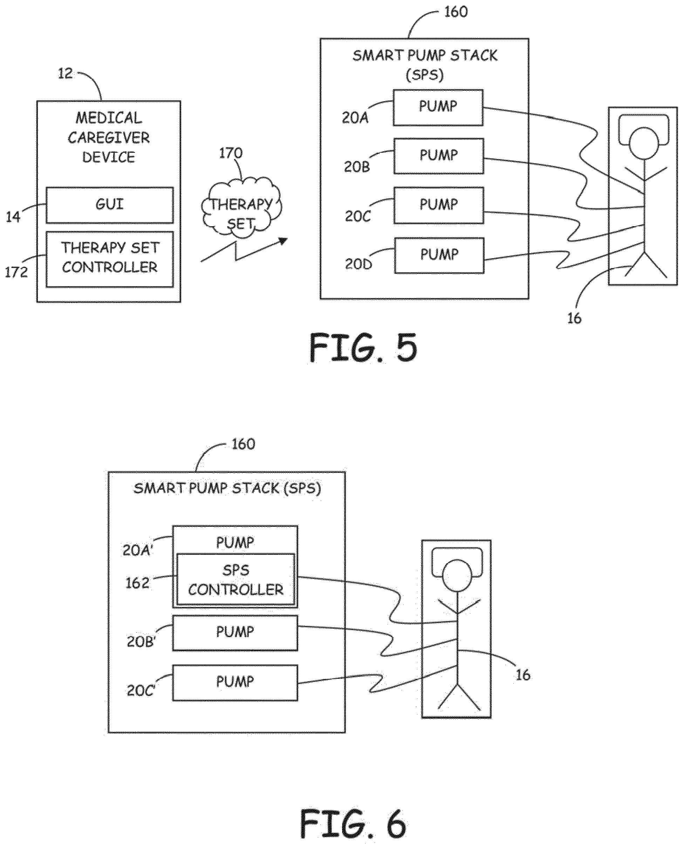

Referring to Fig. 5, one potential environment in which infusion planning systems can be utilized is in a neonatal intensive care unit (NICU). Often in the NICU there are multiple infusion pumps 20 connected to a single infant patient 16. One frequent issue for these types of patients 16 is the need to manage total volume delivered by all devices on an hourly or regular basis. Management of this information is often particularly complicated and time consuming for caregivers.

In a common scenario, there might be six to eight pumps 20 on one patient 16. The total hourly volume maximum might be three to five milliliters total for all pump infusing, for example. One or more of the infusions is likely adjusted frequently based on blood pressure, heart rate, or other parameters. Moreover, when one of the infusions is adjusted, all the rest may require adjustment as well. Knowing which pumps 20 should and should not be adjusted is important to safely and effectively make changes in medical care. Further, each time a change occurs, charting of the information is necessary. All of these necessary and critical steps can be time-consuming and difficult, potentially resulting in significant inefficiencies and medication errors.

Accordingly, embodiments can include an infusion planning system, as described herein that allows all the pumps 20 on one patient 16 to feed information to a centralized GUI 14 for the patient 16. The centralized GUI 14 allows a user to see individual data of each pump 20 and the aggregate totals of all pumps. This information can be conveyed on a GUI similar to the ones shown in Figures 2 and 3, for example. In some embodiments, users can designate which pumps and/or protocols take precedence (or are more important than other pumps/protocols) and accordingly, identify which pumps and protocols should not be changed versus those that can be readily changed. The GUI 14 provides a graphical way for the caregiver to safely experiment with possible changes and see what the impacts are to total volume delivered prior to making changes. As such, the infusion planning system provides a valuable clinical decision support function. Total volume can be understood based on the total infusion bar 120 shown in Figures 2 and 3, for example. This process can be automated in the sense that changes can happen to each pump 20 based on changes to the GUI 14. Auto charting functionality associated with this application is made possible such that nurses are relieved from having to chart each pump change.

### Smart Pump Stacks

As depicted in Figure 5, the infusion planning system may utilize a "Smart Pump Stack" (SPS) 160. In some embodiments, a SPS 160 could be a set of pumps (i.e. pumps 20A, 20B, 20C, and 20D) delivering fluid or therapy to a single patient 16. The pumps in a SPS 160 could be connected and communicating in a way that provides clinical benefit to the patient 16 and/or improved work flow for the medical caretaker.

A SPS 160 can utilize an interface for control/interaction with the medical caregiver device 12 (constituting an SPS controller). The medical caretaker device/SPS controller 12 could be a PC, tablet PC, smart phone, a custom controller designed specifically for use with the SPS 160. Alternatively, a pump 20A' in the SPS 160 could be designated to act as the SPS controller 162 and be used to control and monitor the other pumps (i.e. pumps 20B' and 20C') in the SPS 160, as depicted in Figure 6.

In some embodiments, therapy protocols are created from a set of medication parameters. For example, if a patient required hydration, pain control, antibiotics and blood pressure control infusions delivered by four separate pumps 20A, 20B, 20C, and 20D, the programming for these four fluid infusions could be combined into a therapy set 170 that could be sent to a SPS 160 resulting in the four pumps being programmed at once. Pumps associated with a therapy set 170 could work together such that across all pumps in the therapy set 170, the clinician could see the total fluid that will be delivered per hour to the patient via a GUI 14 on a medical caregiver device 12. Further, when changing a delivery parameter or delivery parameters of a pump 20 in the therapy set 170 the clinician could see the impact on total fluid delivered per hour. The total infusion bar 120 of Figs. 2 and 3 is one example of how this information can be visually conveyed.

A therapy set controller 172 can be incorporated into the medical caregiver device 12 and its programming as shown in Fig. 5. In some embodiments, when changing a delivery parameter or delivery parameters of one pump 20 in the therapy set 170, the therapy set controller 172 could reduce a delivery parameter or delivery parameters of a lower priority drug in the therapy set 170 so as not to exceed an hourly maximum delivery. In some embodiments, the therapy set controller 172 can warn the clinician if delivering a bolus will exceed a time-based, e.g., hourly, maximum delivery. Some embodiments can permit all pumps in the therapy set 170 to be started, paused and stopped at the same time. In certain embodiments, all pumps 20 in the therapy set 170 are assigned relative priority based on the criticality of the drug being delivered or other clinical criteria. In some embodiments, patient data such as patient weight, for example, is entered in the medical caregiver device/SPS controller 12 and is used by all pumps using the therapy set 170. Some embodiments will permit the event logs for all pumps 20 using the therapy set 170 to be combined into a single record.

Pumps 20 using the therapy set 170 are programmed to deliver sequentially in certain embodiments. For example, the therapy set 170 could program the system to first deliver from pump 20A then transition to pump 20B with the same drug when the reservoir of pump 20A is empty. The feature could also be used as a backup in case of problems with pump 20A. In another example, the therapy set 170 could be programmed to deliver from pump 20A then flush with pump 20B when the reservoir of Pump 20A is empty. In another example, pump 20A in the therapy set 170 could be designated as a backup to pump 20B in the therapy set 170. If pump 20B detects an occlusion, error code, depleted battery etc. pump 20A could take over delivery. If pump 20B is stopped by the clinician (to change the syringe, for example) pump 20A could take over until pump 20B is restarted.

In some embodiments, hard or soft limits of pump 20A in a therapy set 170 could be adjusted based on the drug that pump 20B is delivering or the rate at which that drug is being delivered. Various indications for such parameters could be graphically incorporated into a GUI display 14, like those shown in Figs. 2 and 3.

The pumps of a therapy set 170 can be set up to function in a coordinated way in various embodiments. Other pumps operating under a therapy set 170 could be programmed to reduce or stop delivery if a fault occurs in another pump. For example, if two drugs should always be delivered together and one of the pumps stops delivery, the other pump could slow or stop delivery. In other embodiments, all pumps in a therapy set 170 could be set to alarm if one is not responding. Further, in some embodiments, lower priority pumps could share battery power with higher priority pumps.

In some embodiments, the medical caregiver device/SPS controller 12 could "clone" a pump 20 in the SPS 160. If pump 20A faults or needs to be taken out of service, pump 20C, that is in the SPS 160 but not being used, could receive the full programming and current status of pump 20A. All programming and status parameters could be transferred to pump 20C, including moving the pump's drive rod to the correct location. A syringe or other reservoir could be quickly moved from pump 20A to pump 20C with very little delay in therapy and no loss of data. A location in the SPS 160 could be designated as the spare pump spot. A fault in any other pump in the SPS 160 could cause the medical caregiver device/SPS controller 12 to program the spare pump so that it can be quickly swapped with the pump that has faulted.

### Infusion Planner System

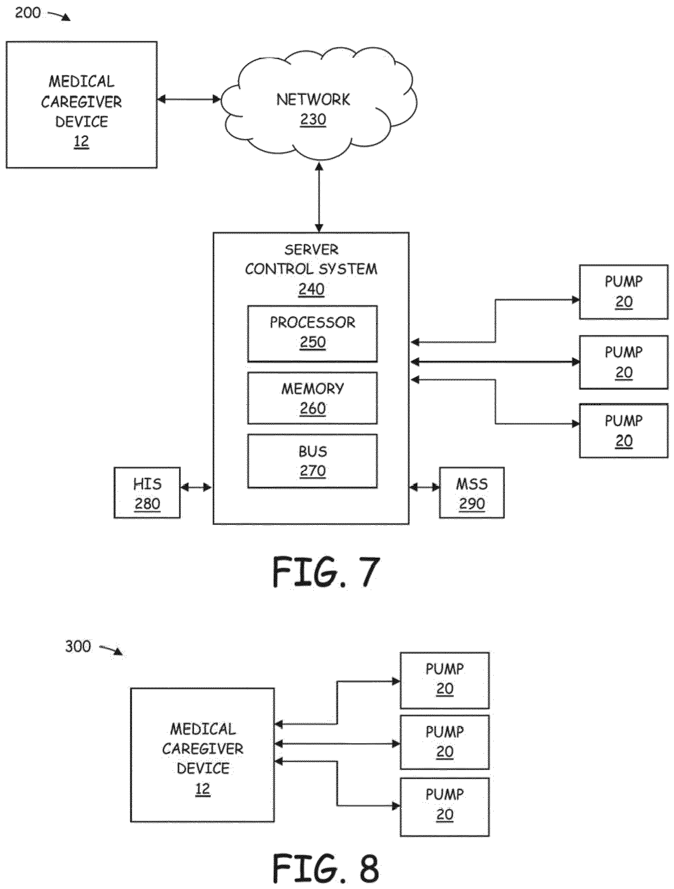

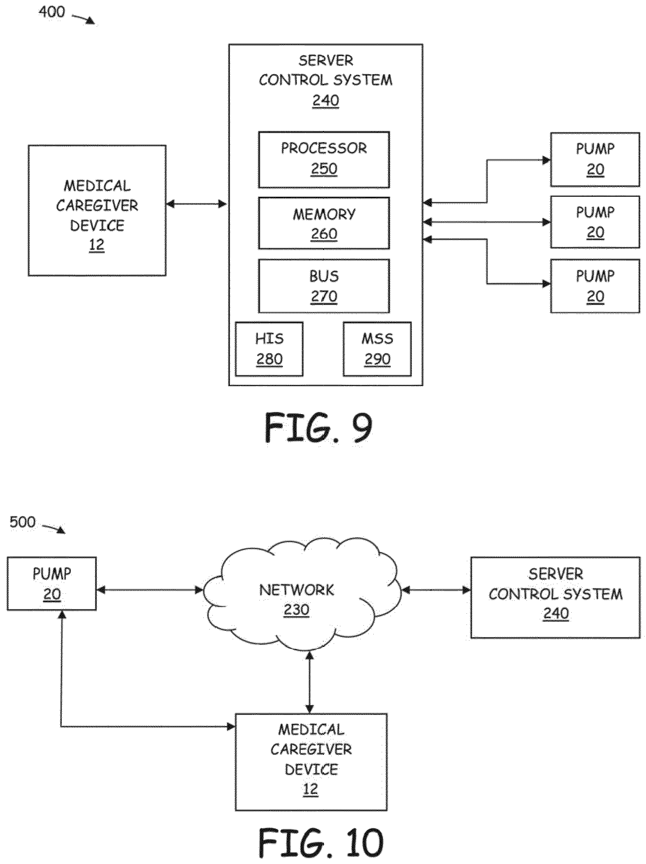

Accordingly, embodiments can provide caregivers with an easy to use system than can be used to plan, coordinate, and monitor medication deliveries and other medical events. While the GUI 14 of Figures 2 and 3 shows one component of the planning tool, the tool typically takes advantage of a larger system similar to of the ones set forth in Figures 7-10. Figures 7-10 each depict examples of possible infusion planning systems 200, 300, 400 and 500 respectively.

### Medical Caregiver Devices

The medical caregiver devices 12, described in Figures 7-10 and throughout this document, can comprise a personal computer, PC workstation, laptop, electronic tablet, smart phone, custom controller, server computer, hand held device or other display and processing device providing a GUI 14. The devices 12 can operate with other general purpose computer systems or computer configurations. These medical caregiver devices 12 can be controlled via a keyboard, mouse, or other touch-less or touch-based input devices. Further, inputs can be speech/voice activated or motion activated as well. Personal computers or PC workstations, for example, might be set up at a hospital unit, nurse station, or patient bedside.

### Pumps

Pumps 20, described in Figures 7-10 and throughout this document, can include a variety of medical infusion pumps. These infusion pumps 20 can include, but are not limited to, peristaltic pumps and syringe pumps, for example. These infusion pumps 20 generally can be used to provide fluids, medication, or nutrition to a patient 16. Infusions made possible can include but are not limited to therapeutic agents; nutrients; drugs; medicaments such as antibiotics, blood clotting agents, and analgesics; and other fluids. The pumps 20 can be used to introduce the medications or fluids into the body of a patient utilizing any of several routes such as, for example, intravenously, subcutaneously, arterially, or epidurally. Infusions can be delivered according to various delivery profiles, such as continuous, intermittent, or patient controlled, for example.

### Network

The network 230, described in the figures and throughout this document, utilized by the system can represent a networking environment such as a local area network or wide area network. In a network environment, programming can be stored in the server memory, one or more medical caregiver devices, or other networked component. The network 230 and server enable connection of devices throughout a hospital, medical facility, research environment, laboratory, clinic, administrative offices, or other connection.

### Server Control System

The server control system 240, described in the figures and throughout this document, is part of a computing environment and can be considered a general purpose computing device in various embodiments. The server control system 240 can include at least a processor 250, memory 260 and data bus 270, for example. Although the many components are generally shown as residing on a single server or computing device, it should be understood that any number of components can reside on any number of servers or computing devices.

### Processor

The processor 250 described in the figures and throughout this document can be any programmable device that accepts digital data as input, is configured to process the input according to instructions or algorithms, and provides results as outputs, for example. In an embodiment, processor 250 can be a central processing unit (CPU) configured to carry out the instructions of a computer program. Processor 250 is therefore configured to perform basic arithmetical, logical, and input/output operations.

### Memory

The memory 260 described in the figures and throughout this document can comprise volatile or non-volatile memory as required by the coupled processor 250 to not only provide space to execute the instructions or algorithms, but to provide the space to store the instructions themselves. In embodiments, volatile memory can include random access memory (RAM), dynamic random access memory (DRAM), or static random access memory (SRAM), for example. In embodiments, non-volatile memory can include read-only memory, flash memory, ferroelectric RAM, hard disk, floppy disk, magnetic tape, or optical disc storage, for example. The foregoing lists in no way limit the type of memory that can be used, as these embodiments are given only by way of example and are not intended to limit the scope of the claims.

### DATA BUS

The data bus 270 described in the figures and throughout this document manages various parts of the systems described and generally serves as a connection framework for various parts, including the processor and memory. In general, the data bus 270 provides a communications architecture for exchanging information throughout the system. The system data bus 270 can include a memory bus, memory controller, peripheral bus or local bus of various bus architectures. These bus architectures can include, but are not limited, to Industry Standard Architecture (ISA), Extended Industry Standard Architecture (EISA), IBM Micro Channel, VESA Local bus, Peripheral Component Interconnect and others.

### HIS

The Hospital Information System (HIS) 280, described in the figures and throughout this document, comprises the information or management system of a hospital, with all of its subcomponents and subsystems. The HIS 280 refers to a system providing healthcare related information that is integrated and is accessible by persons at a hospital or healthcare facility to assist in providing patient care. These are comprehensive, integrated information systems designed to manage the medical, administrative, financial and legal aspects of a hospital and its service processing. The HIS 280 can include or manage electronic medical records for patients. Such electronic records can include up-to-date medical histories, patient data, lab work, test results, prescriptions, imaging and diagnosis information for patients. The HIS 280 can be configured to transmit data to a server for integration into the drug libraries in some embodiments. Likewise, data can be transmitted from a server to the HIS 280 for informational, reporting, or patient care purposes.

### MSS

The Medication Safety Software (MSS) 290 described in the figures and throughout this document includes medication information parameters and drug libraries that can be used by "smart" infusion pumps and medical equipment to assist in safely controlling the introduction of fluids into a patient when medical personnel are not continuously present. MSS 290 information can provide information to smart pumps concerning, or imposing, safety limits on medication program parameters such as dose, concentration, and time, etc., for delivery of a particular medication from the pump to a particular patient. Practitioners create and maintain so-called "drug libraries" associated with such safety limits that are utilized by the MSS 290.

### Methods

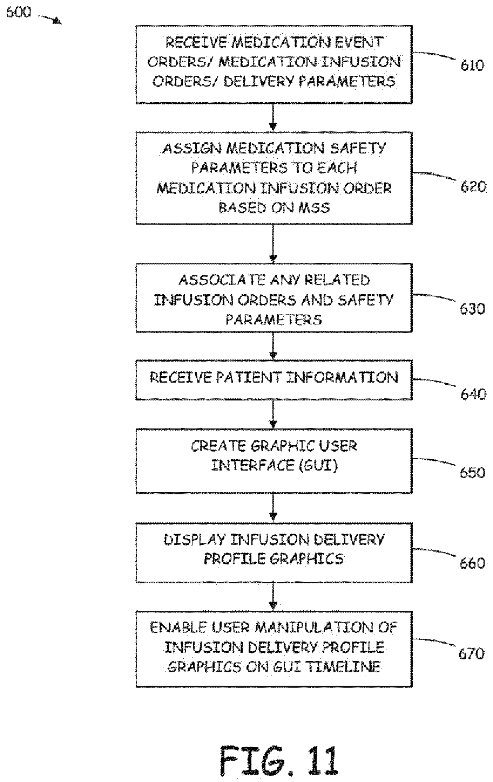

Figure 11 is a flow diagram of an infusion planning method 600 for scheduling infusion and medication delivery. In general, as depicted at 610, the system receives medication event orders, medication infusion orders and delivery parameters. This information can take on a variety of forms, but is generally first accumulated so as to provide some initial order data from which to begin scheduling. At 620, the medication safety parameters are assigned to each medication infusion order based on the requirements of the medication safety software. This is potentially important to ensuring that infusions are safely coordinated and delivered. At 630, any related infusion order and safety parameters are associated. Patient information is also received in some embodiments at 640. Using the information received, a graphic user interface is created. Infusion delivery profile graphics are displayed at 660 and a user is enabled to manipulate the infusion delivery profile at 670.

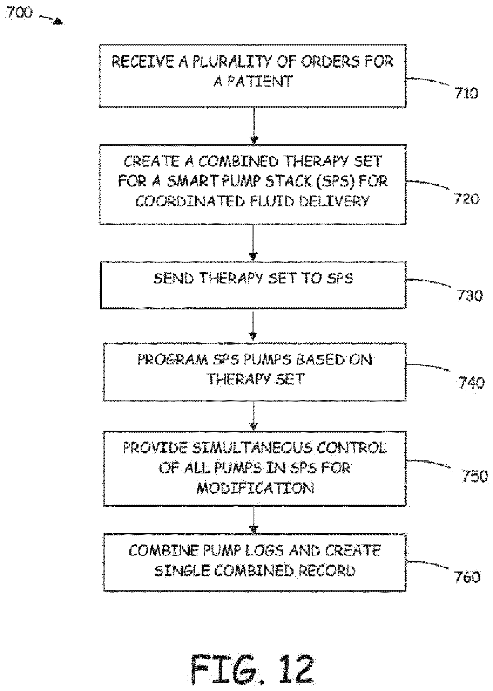

Figure 12 is a flow diagram of an infusion planning method 700 for scheduling infusion and medication delivery. Specifically, the method relates to programming a set of pumps in a smart pump stack (SPS) for coordinated infusion to a single patient. First, activity at 710 relates to receiving a plurality of orders for a single patient. Orders generally relate to medication event orders, medication infusion orders, or delivery parameters associated with different pumps. Activity at 720 relates to creating a combined therapy set for a SPS to enable coordinated fluid delivery between the pumps in the SPS. The therapy set can be created with the assistance of a therapy set controller, for example. The coordinated fluid delivery may be simultaneous, sequential or coordinated otherwise. Activity at 730 relates to sending the therapy set to the SPS. At 740 the SPS pumps are programmed based on the therapy set sent. This programming can be done simultaneously at each of the pumps in some embodiments. At 750 simultaneous control of each of the pumps in the SPS is made possible when modifications are necessary. Finally, in some embodiments the method allows combining the pump logs of each of the pumps in the SPS to create a single combined record at 760. Various methods involving SPS and coordinated fluid delivery can include some or all of the described activities in various orders. Activities can be omitted or others added as well.

### Scalability and Filtering of the Display and Infusion Records

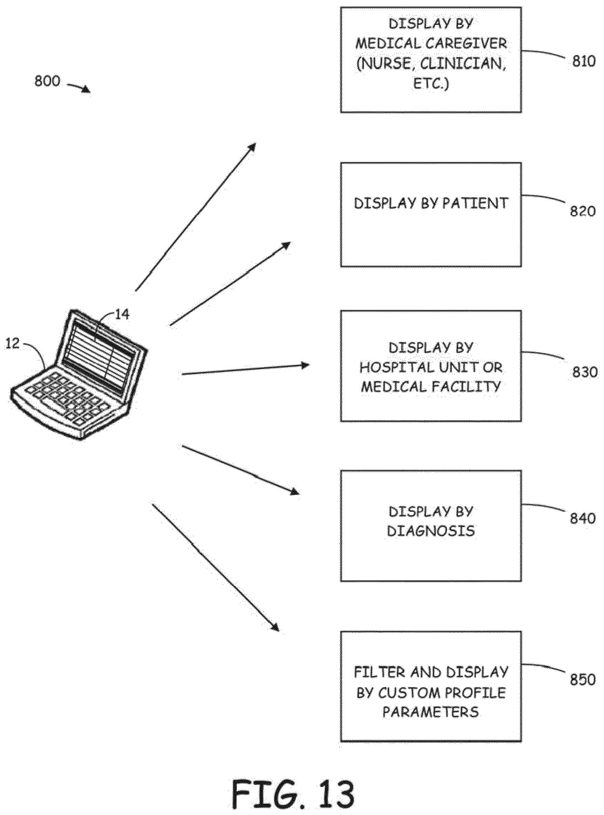

Figure 13 depicts various ways in which the display can be configured to different groups or populations of a different scale to suit desired various applications. A graphic 800 depicting various ways for display of pump, hospital, and patient data on the GUI 14 of a medical caregiver workstation 12 are set forth in this figure. Each of these displays provides a different reference point for organization, scheduling, and optimizing treatment and can, accordingly, be filtered and/or scaled to a display or group of records that best suits the needs of the user. One option for display 810 shown includes a display of infusion information/patient information by medical caregiver. This can be tailored to a specific nurse, clinician, doctor, or medical staff member. Another potential display 820 would display only the data for a specific patient 16 on the GUI 14. This can be useful for a more complete understanding of the needs of one individual patient. Another potential display 830 would display all infusions, pumps and information at the level of hospital unit or at the level of an entire hospital. A display of this scale can be especially useful for tracking the use of hospital resources and could assist a hospital or medical unit to better understand the pumps and other resources likely needed for effective patient care in the future. Another display option 840 would present infusion/medical information based upon a particular diagnosis. Having this data as a reference point would likely enable a nurse, doctor or clinician to more rapidly plan a particular set of infusions and other procedures by looking at past cases handled under similar circumstances.

Similarly, a customized display or filter of various parameters 850 of infusion records of patients meeting a particular profile is possible as well. Specifically, such a display or filter can be used to narrow a group of records to ones having recorded infusion treatments of interest to a physician, nurse, or clinician at their request. These records of past treatments can be used to quickly determine a course of treatment for a patient based on a similar profile. For example, a physician could customize a filter (via a fielded, Boolean or customized search, for example) of patient records to determine what he/she has personally done in the past, for what types of conditions he/she has previously given a particular drug, or what type of treatment has he/she previously given someone with the same age, weight and condition. Quickly reviewing a set of similar, particularly-relevant treatment records associated with a similar patient profile can be of enormous benefit as a reference point for a physician or medical professional in certain circumstances. Greater continuity of best practices and accepted care can be achieved by most medical practitioners through access to such information as well.

In general, the capabilities and usefulness of the type of treatment planning and recording system discussed in this application are greatly enhanced once a large number of patient records of past infusions are compiled in a searchable database. This is made possible, in part, by the easily searchable and reviewable electronic data and common timeline from which patient records of infusions can be drawn with a search or filtering tool. Records can be filtered by treatment characteristics or patient characteristics such as age, weight, gender, ethnicity, care area, treating physician, drug type (opioid, sedative, anti-infective, etc.), drug, number of treatments (i.e. someone on chemo, pain, anti-emetic), procedure code, ICD-9 (primary, secondary, tertiary, etc.), ICD-10, or any combination of these and other parameters documented in the treatment history of a patient. Physiological measurements documented such as blood pressure, heart rate, CO2 and O2 levels, ECG, BIS (Bispectral Index), or pain response meter that could also be patient clinical parameters that could be used as search/filter variables as well. As previously alluded to, the care areas of a hospital assigned to particular drugs commonly used in those areas could also represent a parameter by which a physician, nurse or clinician could filter and or distinguish previous patient records as well. For example, care areas assigned might include any one or more of Adult vs. Children's, Cardiac and Renal vs. Adult general, or ICU vs. PACU. Further, when there are meaningful distinctions between care areas the system could create or make use of suitable parameters to distinguish between those care areas.

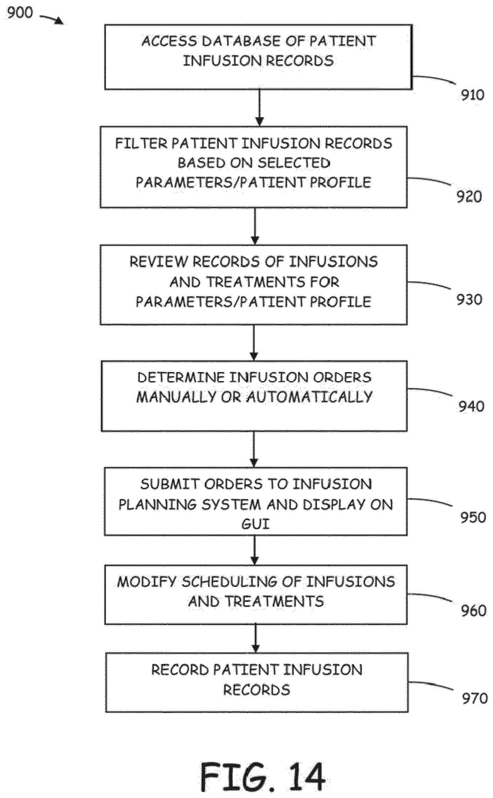

Accordingly, in some embodiments, scheduling of medical treatments, including infusions for a patient, can begin by providing physician, nurses, or clinicians search and filtering tools of past treatment records so they can determine which infusion orders are desired. These records provide information and advice about potential recommended care through a targeted review of past patient records, as set forth in the flow diagram of a method 900 for planning infusion treatments for a patient at a medical facility shown in Fig. 14.

At 910 the physician or medical caregiver accesses a database of timeline-based, graphical patient infusion records. At 920 the physician or medical caregiver filters the graphical patient infusion records with the aid of search software using parameters associated with a patient profile matching those of the patient to locate a set of relevant infusion timeline records. This is done with a search tool to search or filter past compiled patient records based on a number of parameters such as, for example, age, weight, sex, and ICD-9 code. In some embodiments, the physician or medical caregiver will start by filtering to a care area population (i.e. Adult, Children, Cardiac Renal, PACU, CICU) then further filter based on ICD-9 code and drug. In other embodiments, the physician or medical caregiver will filter based on pain medication and location where commonly used and relate to one or more ICD-9 codes. In other embodiments, the physician or medical caregiver will filter based on a combination of specific drugs being used and relate to one or more ICD-9 codes.

The set of relevant infusion timeline records are then reviewed by the physician or medical caregiver at 930 to obtain information related to treatment individuals matching the patient profile. This is done to inform the physician or medical caregiver of past infusions and treatments performed based on this particular profile. At 940, one or more patient infusion orders are determined manually or automatically by the by the physician or medical caregiver in view of the review of the set of relevant records. Automatic determination may provide a recommended computer-generated default that is approved by the by the physician or medical caregiver taking into account the reviewed records. At 950, the patient infusion orders for scheduled treatments are submitted electronically to an infusion planning system responsible for scheduling treatment for the patient within a medical facility, as previously described in this application. The infusion orders for the patient and may be displayed on a GUI 14 as set forth in Figures 2 and 3 in various embodiments.

Further, some activities may or may not be present in various methods. For example, at 960, medical caregivers are able to modify infusion scheduling to optimize the schedule of treatment delivery. Infusion delivery can be modified as necessary, to the extent that the infusion therapies and profiles are not locked by the physician or medical caregiver, or that rules linking and restricting various infusions and parameters is permitted. In certain embodiments, at 970, patient infusion records are recorded. These records can be entered into the database of timeline-based, graphical patient infusion records that may be used by physicians or medical caregivers in the future.

It should also be appreciated that the exemplary embodiment or exemplary embodiments are only examples, and are not intended to limit the scope, applicability, or configuration of the invention in any way. Rather, the foregoing detailed description will provide those skilled in the art with an enabling disclosure for implementing the exemplary embodiment or exemplary embodiments. It should be understood that various changes can be made in the function and arrangement of elements without departing from the scope of the invention as set forth in the appended claims and the legal equivalents thereof.

The embodiments above are intended to be illustrative and not limiting. Additional embodiments are within the claims. Although the present invention has been described with reference to particular embodiments, workers skilled in the art will recognize that changes may be made in form and detail without departing from the spirit and scope of the invention.

Various modifications to the invention may be apparent to one of skill in the art upon reading this disclosure. For example, persons of ordinary skill in the relevant art will recognize that the various features described for the different embodiments of the invention can be suitably combined, un-combined, and re-combined with other features, alone, or in different combinations, within the spirit of the invention. Likewise, the various features described above should all be regarded as example embodiments, rather than limitations to the scope or spirit of the invention. Therefore, the above is not contemplated to limit the scope of the present invention.

For purposes of interpreting the claims for the present invention, it is expressly intended that the provisions of Section 112, sixth paragraph of 35 U.S.C. are not to be invoked unless the specific terms "means for" or "step for" are recited in a claim.

Also provided are the following technology aspects:
1. An infusion planning system for scheduling medical events including medial infusions, comprising:
   a control system including a processor, memory, and data bus that:
      receives a plurality of medication event orders associated with one or more patients including at least a plurality of medication infusion orders for administration by one or more infusion pumps including delivery parameters for each medication infusion order;
      assigns at least one medication safety parameter to each medication infusion order;
      associates any related medication infusion orders and assigned medication safety parameters with one another; and
      receives patient information specific to the one or more patients from a hospital information services database;
   a medical caregiver device operatively coupled to the control system; and
   a graphical user interface (GUI) displayed on the caregiver device including a plurality of infusion delivery profile graphics corresponding to the plurality of medication infusion orders, each of the profile graphics presenting visual information corresponding to both a quantity of medication to be delivered and an amount of time for fluid delivery;
      wherein the GUI includes a schedule timeline permitting user manipulation of the infusion delivery profile graphics on the schedule timeline to assign and revise times for administration of the medication event orders.
2. An infusion planning system according to aspect 1, wherein the medical caregiver device is a PC workstation, a laptop, an electronic tablet, or a smart phone.
3. An infusion planning system according to aspect 1, wherein color-coded scales are provided on the GUI reflecting acceptable fluid delivery ranges for infusions scheduled.
4. An infusion planning system according to aspect 1, wherein the GUI displays a graphic to indicate related medication infusion orders.
5. An infusion planning system according to aspect 1, wherein the GUI displays real-time updates of infusion delivery profile graphics as changes occur over time.
6. An infusion planning system according to aspect 1, wherein the medication infusion orders are not the only medication event orders received by the data bus.
7. An infusion planning system according to aspect 1, wherein the control system controls a smart pump stack comprising a plurality of infusion pumps.
8. A graphical user interface (GUI) for scheduling patient care events including scheduling a plurality of infusions delivered by at least one infusion pump, the GUI comprising:
   a time schedule display comprising a plurality of columns representing time intervals during at least one particular date;
   a plurality of infusion bars each representing an ordered infusion or medication for administration;
   an infusion delivery profile graphic associated with each ordered infusion that depicts both the amount of medication to be delivered to a patient and the length of time period needed for delivery;
   wherein the infusion delivery profile graphics are configured for movement within the infusion bar associated with the corresponding ordered infusion, such that the profile graphic is aligned with the columns representing the time intervals over which the ordered infusion is scheduled for delivery.
9. A graphical user interface (GUI) according to aspect 8, wherein a total infusion bar is included depicting the cumulative total of the ordered infusions during the time intervals displayed on the chart.
10. A graphical user interface (GUI) according to aspect 8, wherein a graphic is used to depict the ordered infusions that are linked to one another.
11. A graphical user interface (GUI) according to aspect 8, wherein real-time updates of changes are shown on the display.
12. A graphical user interface (GUI) according to aspect 8, wherein medical events are displayed for the one or more patients not related to medication infusion therapies.
13. A graphical user interface (GUI) according to aspect 8, wherein a color-coded scale is provided reflecting acceptable ranges of fluid delivery adjacent at least one of the plurality of infusion bars.
14. A non-transitory data storage media, storing computer-usable instructions embodied thereon that cause a computing device to perform a method for scheduling infusion and medication delivery, the method comprising:
   receiving a plurality of medication event orders associated with one or more patients including a plurality of medication infusion orders for administration by one or more infusion pumps including delivery parameters for each medication infusion order;
   assigning medication safety parameters to each medication infusion order;
   associating any related medication infusion orders and assigned medication safety parameters;
   receiving patient information from a hospital information services database specific to the one or more patients;
   creating a graphical user interface, including a display having a plurality of infusion bars set on a common scheduling timeline, each infusion bar associated with a type of medication infusion order and including a first ordinate representative of infusion amount and a second ordinate representative of time;
   displaying an infusion delivery profile graphic for each infusion bar which has a shape that visually depicts the amount of infusion fluid for delivery and the time length of infusion for the medication infusion order associated with that infusion bar; and
   enabling user manipulation of the infusion delivery profile graphic within the infusion bar to schedule the timing for a corresponding medication infusion.
15. The method of aspect 14, wherein a record of actual infusion and medication delivery for each of the one or more patients is made.
16. The method of aspect 15, wherein the display of the graphical user interface is updated in real-time.
17. The method of aspect 14, wherein the graphical user interface includes a total infusion bar depicting cumulative amounts of infusion fluid delivered during an interval of time.
18. A method for planning infusion treatments for a patient at a medical facility, comprising accessing a database of timeline-based, graphical patient infusion records;
   filtering graphical patient infusion records with the aid of search software using parameters associated with a patient profile matching those of the patient to locate a set of relevant infusion timeline records;
   reviewing the set of relevant infusion timeline records to obtain information related to treatment individuals matching the patient profile;
   determining one or more patient infusion orders for the patient in view of the review of the set of relevant records;
   submitting the one or more patient infusion orders electronically to an infusion planning system responsible for scheduling and recording infusion treatments for the patient within the medical facility.
19. The method of aspect 18, wherein the infusion planning system assigns the one or more patient infusion orders specific rules restricting the scheduling of the various infusion orders relative to each other.
20. The method of aspect 19, wherein the parameters include one or more patient characteristics including one or more of: age, weight, gender, ethnicity.
21. The method of aspect 20, wherein the parameters include one or more treatment characteristics including one or more of: ICD-9 code, ICD-10 code, drug type, care area, treating physician, number of treatments, procedure code.
22. The method of aspect 21, wherein the parameters include one or more patient physiological measurements including one or more of: blood pressure, heart rate, CO2 level, O2 level, ECG, BIS, or pain response meter reading.

## Claims

1. An infusion planning system for scheduling medical events including medical infusions, comprising:
a control system including a processor, memory, and data bus that:
receives a plurality of medication event orders associated with one or more patients including at least a plurality of medication infusion orders for administration by one or more infusion pumps including delivery parameters for each medication infusion order;
assigns at least one medication safety parameter to each medication infusion order;
associates any related medication infusion orders and assigned medication safety parameters with one another; and
receives patient information specific to the one or more patients from a hospital information services database; and
a medical caregiver device operatively coupled to the control system, the medical caregiver device including a graphical user interface (GUI) including a planned infusion display portion and delivered infusion display portion,
wherein the planned infusion display portion includes a time schedule having a plurality of columns representing time intervals during at least one particular date, and a plurality of planned infusion delivery profile graphics corresponding to the plurality of medication infusion orders, each of the planned infusion delivery profile graphics presenting visual information corresponding to both a quantity of medication to be delivered and an amount of time for delivery of the medication, and
wherein the delivered infusion display portion includes a time schedule having a plurality of columns representing time intervals during the at least one particular date, and a plurality of delivered infusion graphics corresponding to an actual delivery of medication, each of the delivered infusion graphics presenting visual information corresponding to both a quantity of medication actually delivered and the amount of time of the delivery of the medication, and
wherein the planned infusion delivery profile graphics are configured for movement relative to the columns representing the time intervals to provide a graphical display of infusion time and total volume of medication scheduled to be delivered to at least one of the plurality of patients prior to making changes to the one or more infusion pumps.

2. An infusion planning system according to claim 1, wherein the medical caregiver device is a PC workstation, a laptop, an electronic tablet, or a smart phone.

3. An infusion planning system according to claim 1, wherein color-coded scales are provided on the GUI reflecting acceptable fluid delivery ranges for infusions scheduled.

4. An infusion planning system according to claim 1, wherein the GUI displays:
(a) a graphic to indicate related medication infusion orders; or
(b) real-time updates of infusion delivery profile graphics as changes occur over time.

5. An infusion planning system according to claim 1, wherein the medication infusion orders are not the only medication event orders received by the data bus.

6. An infusion planning system according to claim 1, wherein the control system controls a smart pump stack comprising a plurality of infusion pumps.

7. A graphical user interface (GUI) for scheduling patient care events including scheduling a plurality of infusions delivered by at least one infusion pump, the GUI comprising:
a planned infusion display portion including a time schedule display comprising a plurality of columns representing time intervals during at least one particular date, and a plurality of planned infusion bars each representing an ordered infusion or medication for administration depicting both the amount of medication to be delivered to a patient and the length of time period needed for delivery; and
a delivered infusion display portion including a time schedule display comprising a plurality of columns representing time intervals during at least one particular date, and one or more delivered infusion bars depicting both the amount of the medication actually delivered to the current patient and the length of time of the delivery;
wherein the planned infusion bars are configured for movement relative to the columns representing the time intervals to provide a graphical display of infusion time and medication volume scheduled to be delivered to at least one of the plurality of patients enabling safe evaluation of impacts to total volume delivered for each of the patients prior to making changes, and to enable the coordination of infusion medication orders for the plurality of patients.

8. A graphical user interface (GUI) according to claim 7, wherein a total infusion bar is included depicting the cumulative total of the ordered infusions during the time intervals displayed on the chart.

9. A graphical user interface (GUI) according to claim 7, wherein:
(a) a graphic is used to depict the ordered infusions that are linked to one another;
(b) real-time updates of changes are shown on the display;
(c) medical events are displayed for the one or more patients not related to medication infusion therapies; or
(d) a color-coded scale is provided reflecting acceptable ranges of fluid delivery adjacent at least one of the plurality of infusion bars.

10. A non-transitory data storage media, storing computer-usable instructions embodied thereon that cause a computing device to perform a method for scheduling infusion and medication delivery, the method comprising:
receiving a plurality of medication event orders associated with one or more patients including a plurality of medication infusion orders for administration by one or more infusion pumps including delivery parameters for each medication infusion order;
assigning medication safety parameters to each medication infusion order;
associating any related medication infusion orders and assigned medication safety parameters;
receiving patient information from a hospital information services database specific to the one or more patients;
providing a graphical user interface, including a planned infusion display portion and delivered infusion display portion,
wherein the planned infusion display portion includes a time schedule having a plurality of columns representing time intervals during at least one particular date, and a plurality of planned infusion bars corresponding to the plurality of medication infusion orders and depicting both a quantity of medication to be delivered and an amount of time for delivery of the medication, and
wherein the delivered infusion display portion includes a time schedule having a plurality of columns representing time intervals during the at least one particular date, and a plurality of delivered infusion graphics depicting both a quantity of medication actually delivered and the amount of time of the delivery of the medication, and
wherein the plurality of planned infusion bars are configured for movement relative to the columns representing the time intervals to provide a graphical display of infusion time and total volume of medication scheduled to be delivered to at least one of the plurality of patients prior to making changes to the one or more infusion pumps.

11. The storage media of claim 10, wherein in said method a record of actual infusion and medication delivery for each of the one or more patients is made, preferably wherein the display of the graphical user interface is updated in real-time.

12. The storage media of claim 10, wherein in said method the graphical user interface includes a total infusion bar depicting cumulative amounts of infusion fluid delivered during an interval of time.

13. A method for planning infusion treatments for a patient at a medical facility, comprising
accessing a database of timeline-based, graphical patient infusion records;
filtering graphical patient infusion records with the aid of search software using parameters associated with a patient profile matching those of the patient to locate a set of relevant infusion timeline records;
reviewing the set of relevant infusion timeline records to obtain information related to treatment individuals matching the patient profile;
determining one or more patient infusion orders for the patient in view of the review of the set of relevant records;
submitting the one or more patient infusion orders electronically to an infusion planning system responsible for scheduling and recording infusion treatments for the patient within the medical facility; and
providing a graphical user interface, including a planned infusion display portion and delivered infusion display portion,
wherein the planned infusion display portion includes a time schedule having a plurality of columns representing time intervals during at least one particular date, and one or more planned infusion bars corresponding to the one or more patient infusion orders, each planned infusion bar depicting both the amount of a medication to be delivered to the current patient and the length of time needed for delivery,
wherein the delivered infusion display portion includes a time schedule having a plurality of columns representing time intervals during the at least one particular date, and one or more delivered infusion bars depicting both the amount of the medication actually delivered to the current patient and the length of time of the delivery, and
wherein the one or more planned infusion bars are configured for movement relative to the columns representing the time intervals to provide a graphical display of infusion time and total volume of medication scheduled to be delivered to the current patient.

14. The method of claim 13, wherein the infusion planning system assigns the one or more patient infusion orders specific rules restricting the scheduling of the various infusion orders relative to each other.

15. The method of claim 14, wherein the parameters include one or more patient characteristics including one or more of: age, weight, gender, ethnicity, preferably wherein the parameters include one or more treatment characteristics including one or more of: ICD-9 code, ICD-10 code, drug type, care area, treating physician, number of treatments, procedure code, more preferably wherein the parameters include one or more patient physiological measurements including one or more of: blood pressure, heart rate, CO2 level, O2 level, ECG, BIS, or pain response meter reading.
